(19) Europäisches Patentamt
European Patent Office
Office européen des brevets

(11) EP 1 916 248 A1

(12) EUROPEAN PATENT APPLICATION

(43) Date of publication:
**30.04.2008 Bulletin 2008/18**

(21) Application number: **06122877.1**

(22) Date of filing: **24.10.2006**

(51) Int Cl.:
***C07D 417/12*** *(2006.01)* ***A61K 31/495*** *(2006.01)*
***A61P 35/00*** *(2006.01)* ***A61P 19/00*** *(2006.01)*
***C07K 5/06*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **MEDIVIR AB**
**141 44 Huddinge (SE)**

(72) Inventor: **The designation of the inventor has not yet been filed**

(74) Representative: **Teuten, Andrew John**
**Sagittarius Intellectual**
**Property Consultants Ltd**
**Taylor House**
**39 High Street**
**Marlow, Bucks. SL7 1AF (GB)**

# (54) Furanone derivatives as cysteine protease inhibitors

(57) Compounds of the Formula II

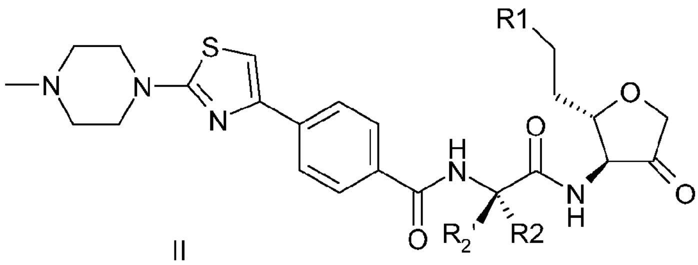

II

wherein
$R^1$ is H, OH, F
$R^2$ is $-CH_2CH(CH_3)_2$, $-CHC(CH_2)_3$ or $CH(CH_3)(CH_2CH_3)$;
$R^{2'}$ is H; or
$R^2$ and $R^{2'}$ together with the adjacent C atom defines cyclohexyl;
and pharmaceutically acceptable salts, hydrates and N-oxides thereof have utility in the treatment of disorders characterised by inappropriate expression or activation of cathepsin K, such as osteoporosis, osteoarthritis, rheumatoid arthritis or bone metastases.

EP 1 916 248 A1

## Description

Field of the invention.

This invention relates to inhibitors of cysteine proteases, especially those of the papain superfamily. The invention provides novel compounds useful in the prophylaxis or treatment of disorders stemming from misbalance of physiological proteases such as cathepsin K.

Description of the related art.

The papain superfamily of cysteine proteases is widely distributed in diverse species including mammals, invertebrates, protozoa, plants and bacteria. A number of mammalian cathepsin enzymes, including cathepsins B, F, H, K, L, O and S, have been ascribed to this superfamily, and inappropriate regulation of their activity has been implicated in a number of metabolic disorders including arthritis, muscular dystrophy, inflammation, glomerulonephritis and tumour invasion. Pathogenic cathepsin like enzymes include the bacterial gingipains, the malarial falcipains I, II, III et seq and cysteine proteases from *Pneumocystis carinii, Trypanosoma cruzei* and *brucei, Crithidia fusiculata, Schistosoma* spp.
The inappropriate regulation of cathepsin K has been implicated in a number of disorders including osteoporosis, gingival diseases such as gingivitis and periodontitis, Paget's disease, hypercalcaemia of malignancy and metabolic bone disease. In view of its elevated levels in chondroclasts of osteoarthritic synovium, cathepsin K is implicated in diseases characterised by excessive cartilage or matrix degradation, such as osteoarthritis and rheumatoid arthritis. Metastatic neoplastic cells typically express high levels of proteolytic enzymes that degrade the surrounding matrix and inhibition of cathepsin K may thus assist in treating neoplasias.
International patent application no WO02/88106 discloses inter alia the compound:

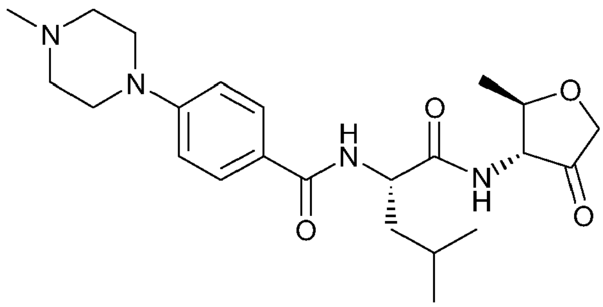

The compounds are suggested as useful in the treatment of disorders in which cathepsin K is implicated.
We have now discovered that elongation at each end of the molecule produces a further group of furanone inhibitors of cathepsin K tending to have with superior efficacy and pharmacokinetic performance.

Brief description of the invention

In accordance with the invention, there is provided compounds of the Formula II

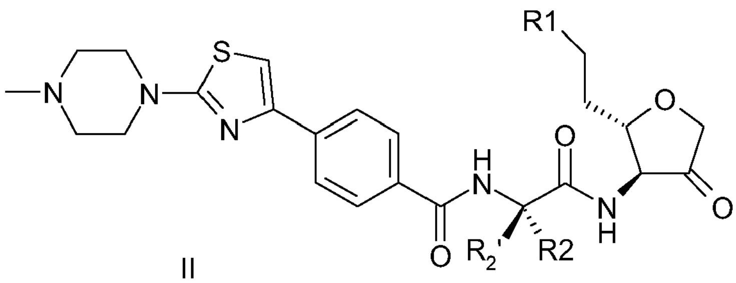

II

wherein

$R^1$ is H, OH, F
$R^2$ is $-CH_2CH(CH_3)_2$, $-CH_2C(CH_3)_3$ or $-CH(CH_3)(CH_2CH_3)$;
$R^{2'}$ is H; or
$R^2$ and $R^{2'}$ together with the adjacent C atom defines cyclohexyl;
and pharmaceutically acceptable salts, hydrates and N-oxides thereof.

Intriguingly, the compounds of the invention appear to augment matrix derived growth factor release by human osteoclasts as described in Fuller, Lawrence Ross, Grabowska Shiroo, Samuelsson and Chambers in an eponymous article simultaneously submitted to J. Clin. Invest. and incorporated by reference herein.

One currently preferred embodiment of formula II comprises the structure:

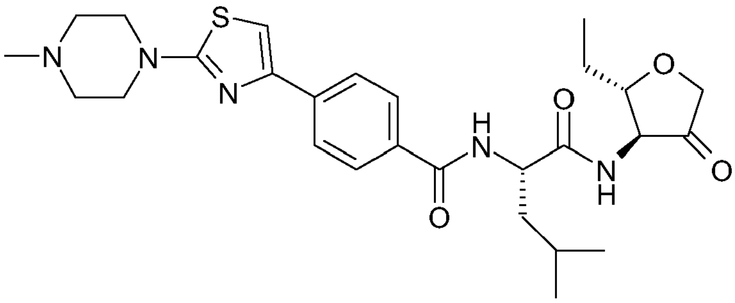

and pharmaceutically acceptable salts, hydrates and N-oxides thereof.

An alternative currently preferred embodiment comprises compounds of the formula:

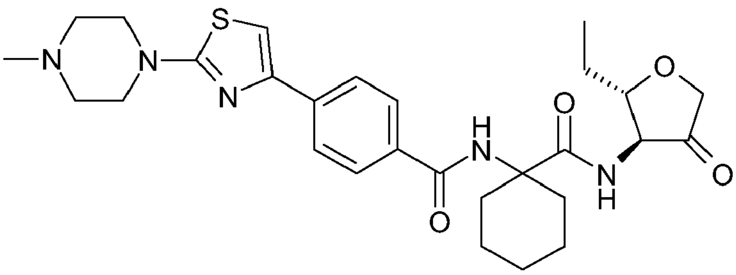

and pharmaceutically acceptable salts, hydrates and N-oxides thereof.

Additional aspects of the invention include a pharmaceutical composition comprising a compound as defined above and a pharmaceutically acceptable carrier or diluent therefor.

A further aspect of the invention is the use of a compound as defined above in the manufacture of a medicament for the treatment of disorders mediated by cathepsin K, such as:

osteoporosis,
gingival diseases such as gingivitis and periodontitis,
Paget's disease,
hypercalcaemia of malignancy
metabolic bone disease
diseases characterised by excessive cartilage or matrix degradation, such as osteoarthritis and rheumatoid arthritis,
bone cancers including neoplasia,
pain.

The invention is believed to be of particular utility against osteoporosis, osteoarthritis, rheumatoid arthritis and/or bone metastases.

Pharmaceutical compositions of the invention typically comprise a high enantiomeric ratio of the furanone stereochemistry depicted in Formula II, such as in excess of 80%, preferably at least 90% (e.g. at least 95%) of the depicted epimer over other epimers at the C-4 centre. However, enantiomeric mixtures below these preferred degrees of ee are within the scope of the invention.

It will be appreciated that the furanone moiety can exist in alternative forms, such as hydrates:

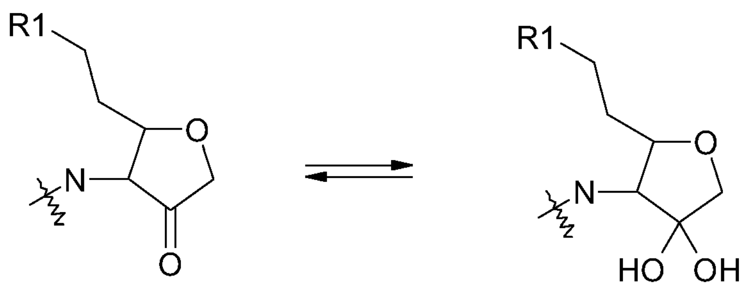

and the invention extends to all such alternative forms.

The compounds of the invention can form salts which form an additional aspect of the invention. Appropriate pharmaceutically acceptable salts of the compounds of Formula II include salts of organic acids, especially carboxylic acids, including but not limited to acetate, trifluoroacetate, lactate, gluconate, citrate, tartrate, maleate, malate, pantothenate, isethionate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succinate, organic sulphonic acids such as methanesulphonate, ethanesulphonate, 2-hydroxyethane sulphonate, camphorsulphonate, 2-napthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids. The compounds of Formula II may in some cases be isolated as the hydrate. Hydrates are typically prepared by recrystallisation from an aqueous/organic solvent mixture using organic solvents such as dioxin, tetrahydrofuran or methanol.

The N-oxides of compounds of Formula II can be prepared by methods known to those of ordinary skill in the art. For example, N-oxides can be prepared by treating an unoxidized form of the compound of Formula (II) with an oxidizing agent (e.g., trifluoroperacetic acid, permaleic acid, perbenzoic acid, peracetic acid, meta-chloroperoxybenzoic acid, or the like) in a suitable inert organic solvent (e.g., a halogenated hydrocarbon such as dichloromethane) at approximately 0°C. Alternatively, the N-oxides of the compounds of Formula (II) can be prepared from the N-oxide of an appropriate starting material.

Compounds of Formula (II) in unoxidized form can be prepared from N-oxides of compounds of Formula (II) by treating with a reducing agent (e.g., sulfur, sulfur dioxide, triphenyl phosphine, lithium borohydride, sodium borohydride, phosphorus bichloride, tribromide, or the like) in an suitable inert organic solvent (e.g., acetonitrile, ethanol, aqueous dioxane, or the like) at 0 to 80°C.

Compounds of Formula (II) can be prepared as their individual stereoisomers by reacting a racemic mixture of the compound with an optically active resolving agent to form a pair of diastereoisomeric compounds, separating the diastereomers and recovering the optically pure enantiomer. While resolution of enantiomers can be carried out using covalent diasteromeric derivatives of compounds of Formula (II), dissociable complexes are preferred (e.g., crystalline; diastereoisomeric salts). Diastereomers have distinct physical properties (e.g., melting points, boiling points, solubilities, reactivity, etc.) and can be readily separated by taking advantage of these dissimilarities. The diastereomers can be separated by chromatography, for example HPLC or, preferably, by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from their racemic mixture can be found in Jean Jacques Andre Collet, Samuel H. Wilen, Enantiomers, Racemates and Resolutions, John Wiley & Sons, Inc. (1981).

It will be appreciated that the invention extends to prodrugs, solvates, complexes and other forms releasing a compound of Formula II in vivo.

While it is possible for the active agent to be administered alone, it is preferable to present it as part of a pharmaceutical formulation. Such a formulation will comprise the above defined active agent together with one or more acceptable carriers/excipients and optionally other therapeutic ingredients. The carrier(s) must be acceptable in the sense of being compatible with the other ingredients of the formulation and not deleterious to the recipient.

The formulations include those suitable for rectal, nasal, topical (including buccal and sublingual), vaginal or parenteral (including subcutaneous, intramuscular, intravenous and intradermal) administration, but preferably the formulation is an orally administered formulation. The formulations may conveniently be presented in unit dosage form, e.g. tablets and sustained release capsules, and may be prepared by any methods well known in the art of pharmacy.

Such methods include the step of bringing into association the above defined active agent with the carrier. In general, the formulations are prepared by uniformly and intimately bringing into association the active agent with liquid carriers or finely divided solid carriers or both, and then if necessary shaping the product. The invention extends to

methods for preparing a pharmaceutical composition comprising bringing a compound of Formula II or its pharmaceutically acceptable salt in conjunction or association with a pharmaceutically acceptable carrier or vehicle. If the manufacture of pharmaceutical formulations involves intimate mixing of pharmaceutical excipients and the active ingredient in salt form, then it is often preferred to use excipients which are non-basic in nature, i.e. either acidic or neutral.

Formulations for oral administration in the present invention may be presented as discrete units such as capsules, cachets or tablets each containing a predetermined amount of the active agent; as a powder or granules; as a solution or a suspension of the active agent in an aqueous liquid or a nonaqueous liquid; or as an oil-in-water liquid emulsion or a water in oil liquid emulsion and as a bolus etc.

With regard to compositions for oral administration (e.g. tablets and capsules), the term suitable carrier includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring or the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

The appropriate dosage for the compounds or formulations of the invention will depend upon the indication and the patient and is readily determined by conventional animal trials. Dosages providing intracellular (for inhibition of physiological proteases of the papain superfamily) concentrations of the order 0.01-100 uM, more preferably 0.01-10 uM, such as 0.1-25 uM are typically desirable and achievable. Solid dosage units of the invention will typically comprise tablets or capsules of 200 - 1500 mg, comprising in the case of tablets 50-95%, preferably 60-75% actives and the remainder excipients as discussed above.

Compounds of the invention are prepared by a variety of solution and solid phase chemistries.

The compounds are typically prepared as building blocks reflecting the P1, P2 and P3 moieties of the end product inhibitor. Without in any way wishing to be bound by theory, or the ascription of tentative binding modes for specific variables, the notional concepts P1, P2 and P3 as used herein are provided for convenience only and have substantially their conventional Schlecter & Berger meanings and denote those portions of the inhibitor believed to fill the S1, S2, and S3 subsites respectively of the enzyme, where S1 is adjacent the cleavage site and S3 remote from the cleavage site. Compounds defined by Formula II are intended to be within the scope of the invention, regardless of binding mode.

Broadly speaking the P1 building block will typically be an intermediate of the formula:

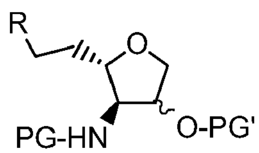

where R is H, F, OH or O-PG" where PG" is a hydroxyl protecting group, PG is H or an N-protecting group and PG' is H, a hydroxyl protecting group or together with the adjacent oxygen defines a keto group. Such intermediates are extensively described in WO2006/64286.

The P2 building block will typically be an N-protected: L-leucine, L-tertiary leucine or 1-amino-cyclohexane carboxylic acid, all of which are commercially available, wherein the N-protecting group is conveniently CBz, Boc or Fmoc.

Preparation of the P3 building block is exemplified below.

Formation of a peptide bond, ie coupling can be carried out using standard coupling procedures such as the

azide method, mixed carbonic-carboxylic acid anhydride (isobutyl chloroformate) method, carbodiimide (dicyclohexylcarbodiimide, diisopropylcarbodiimide, or water-soluble carbodiimide) method, active ester (pnitrophenyl ester, N-hydroxysuccinic imido ester) method, Woodward reagent K-method, carbonyldiimidazole method, phosphorus reagents or oxidation-reduction methods. Some of these methods (especially the carbodiimide method) can be enhanced by adding 1-hydroxybenzotriazole or 4-DMAP. These coupling reactions can be performed in either solution (liquid phase) or solid phase.

More explicitly, the coupling step involves the dehydrative coupling of a free carboxyl of one reactant with the free amino group of the other reactant in the present of a coupling agent to form a linking amide bond. Descriptions of such coupling agents are found in general textbooks on peptide chemistry, for example, M. Bodanszky, "Peptide Chemistry", 2nd rev ed., Springer-Verlag, Berlin, Germany, (1993) hereafter simply referred to as Bodanszky, the contents of which are hereby incorporated by reference. Examples of suitable coupling agents are N,N'-dicyclohexylcarbodiimide, 1-hydroxybenzotriazole in the presence of N,N'-dicyclohexylcarbodiimide or N-ethyl-N'-[(3dimethylamino) propyl] carbodiimide. A practical and useful coupling agent is the commercially available (benzotriazol-1-yloxy) tris-(dimethylamino) phosphonium hexafluorophosphate, either by itself or in the present of 1-hydroxybenzotriazole or 4-DMAP. Another practical and useful coupling agent is commercially available 2-(IH-benzotriazol-1-yl)-N,N,N',N'- tetramethyluronium tetrafluoroborate. Still another practical and useful coupling agent is commercially available 0-(7-azabenzotrizol-1-yl)-N, N,N',N'-tetramethyluronium hexafluorophosphate.

The coupling reaction is conducted in an inert solvent, e. g. dichloromethane, acetonitrile or dimethylformamide. An excess of a tertiary amine, e.g. diisopropylethylamine, N-methylmorpholine, N-methylpyrrolidine or 4-DMAP is added to maintain the reaction mixture at a pH of about 8. The reaction temperature usually ranges between 0 °C and 50 °C and the reaction time usually ranges between 15 min and 24 h.

The functional groups of the various building blocks generally must be protected during the coupling reactions to avoid formation of undesired bonds. The protecting groups that can be used are listed in Greene, "Protective Groups in Organic Chemistry", John Wiley & Sons, New York (1981) and "The Peptides: Analysis, Synthesis, Biology", Vol. 3, Academic Press, New York (1981), hereafter referred to simply as Greene, the disclosures of which are hereby incorporated by reference.

The alpha-carboxyl group of the C-terminal residue is usually protected as an ester that can be cleaved to give the carboxylic acid. Protecting groups that can be used include 1) alkyl esters such as methyl, trimethylsilyl and t-butyl, 2) aralkyl esters such as benzyl and substituted benzyl, or 3) esters that can be cleaved by mild base or mild reductive means such as trichloroethyl and phenacyl esters.

The alpha-amino group of each amino acid to be coupled is typically N-protected. Any protecting group known in the art can be used. Examples of such groups include: 1) acyl groups such as formyl, trifluoroacetyl, phthalyl, and p-toluenesulfonyl; 2) aromatic carbamate groups such as benzyloxycarbonyl (Cbz or Z) and substituted benzyloxycarbonyls, and 9-fluorenylmethyloxycarbonyl (Fmoc); 3) aliphatic carbamate groups such as tertbutyloxycarbonyl (Boc), ethoxycarbonyl, diisopropylmethoxycarbonyl, and allyloxycarbonyl; 4) cyclic alkyl carbamate groups such as cyclopentyloxycarbonyl and adamantyloxycarbonyl; 5) alkyl groups such as triphenylmethyl and benzyl; 6) trialkylsilyl such as trimethylsilyl; and 7) thiol containing groups such as phenylthiocarbonyl and dithiasuccinoyl. The preferred alpha-amino protecting group is either Boc or Fmoc. Many amino acid derivatives suitably protected for peptide synthesis are commercially available.

The alpha-amino protecting group is typically cleaved prior to the next coupling step. When the Boc group is used, the methods of choice are trifluoroacetic acid, neat or in dichloromethane, or HCl in dioxane or in ethyl acetate. The resulting ammonium salt is then neutralized either prior to the coupling or in situ with basic solutions such as aqueous buffers, or tertiary amines in dichloromethane or acetonitrile or dimethylformamide. When the Fmoc group is used, the reagents of choice are piperidine or substituted piperidine in dimethylformamide, but any secondary amine can be used. The deprotection is carried out at a temperature between 0 °C and room temperature usually 20-22 °C

Once the inhibitor sequence is completed any protecting groups are removed in whatever manner is dictated by the choice of protecting groups. These procedures are well known to those skilled in the art.

The P1 building block is typically elongated with the natural or non natural P2 amino acid (or the P3+P2 building block) by conventional solution or solid phase chemistries, such as those outlined or exemplified below, or disclosed in WO00/69855, WO02/057270 or WO06/64286. The P1-P2-P3 sequence can be built up with the P1 building block as a preformed ketone:

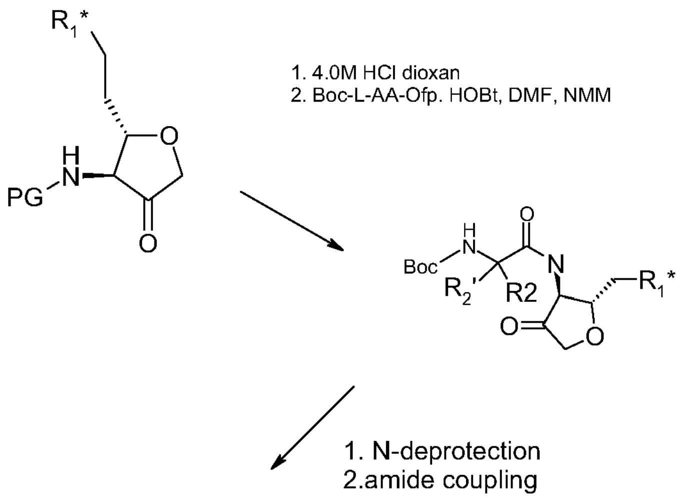


formula II

Scheme 1. Typical elongation of a cyclic ketone

R1* is H, F or protected hydroxyl.

Alternatively, the P1 building block ketone can be transformed to a dimethylketal protection which is then carried through the synthesis and then deprotected in the final step with TFA to give the required ketal.

Alternatively the P1 building block as the hydroxyl may be elongated and subsequently oxidised as shown in Scheme 2.

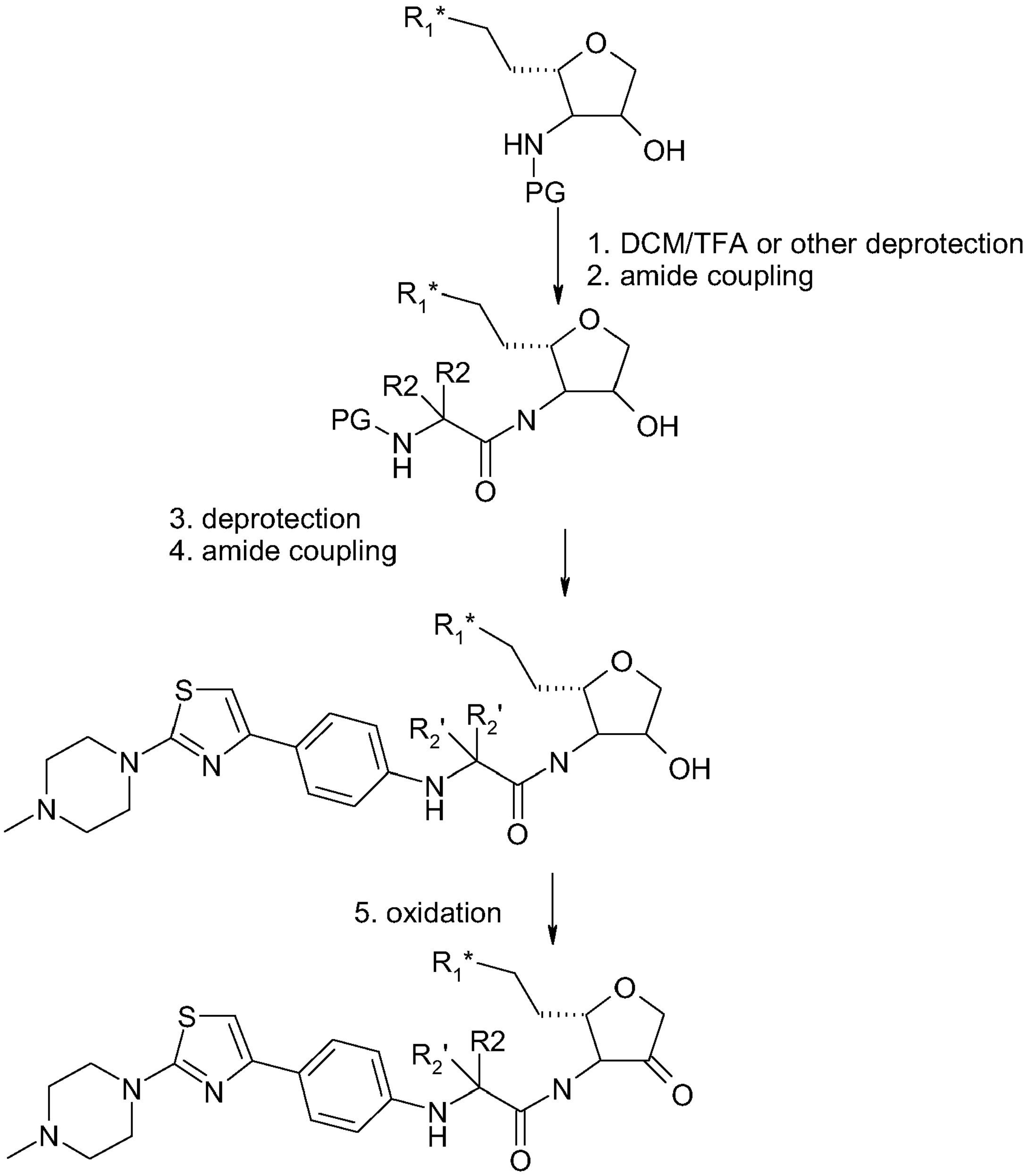


Scheme 14, Typical elongation of an hydroxylated P1 building block

The term "N-protecting group" or "N-protected" as used herein refers to those groups intended to protect the N-terminus of an amino acid or peptide or to protect an amino group against undesirable reactions during synthetic procedures. Commonly used N-protecting groups are disclosed in Greene, "Protective Groups in Organic Synthesis" (John Wiley & Sons, New York, 1981), which is hereby incorporated by reference. N-protecting groups include acyl groups such as formyl, acetyl, propionyl, pivaloyl, t-butylacetyl, 2-chloroacetyl, 2-bromoacetyl, trifluoracetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl, and the like, carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butoxycarbonyl, diisopropylmethoxycarbonyl, isopropyloxycarbonyl, ethoxycarbonyl, methoxycarbonyl, allyloxycarbonyl, 2,2,2-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl, and the like; alkyl gropus such as benzyl, triphenylmethyl, benzyloxymethyl and the like; and silyl groups such as trimethylsilyl and the like. Favoured N-protecting groups include formyl, acetyl, benzoyl, pivaloyl, t-butylacetyl, phenylsulfonyl, benzyl, t-butoxycarbonyl (BOC) and benzyloxycarbonyl (Cbz).

Hydroxy and/or carboxy protecting groups are also extensively reviewed in Greene ibid and include ethers such as methyl, substituted methyl ethers such as methoxymethyl, methylthiomethyl, benzyloxymethyl, t-butoxymethyl, 2-methoxyethoxymethyl and the like, silyl ethers such as trimethylsilyl (TMS), t-butyldimethylsilyl (TBDMS) tribenzylsilyl, triphenylsilyl, t-butyldiphenylsilyl triisopropyl silyl and the like, substituted ethyl ethers such as 1-ethoxymethyl, 1-methyl-1-methoxyethyl, t-butyl, allyl, benzyl, p-methoxybenzyl, dipehenylmethyl, triphenylmethyl and the like, aralkyl groups such as trityl, and pixyl (9-hydroxy-9-phenylxanthene derivatives, especially the chloride). Ester hydroxy protecting groups include esters such as formate, benzylformate, chloroacetate, methoxyacetate, phenoxyacetate, pivaloate, adamantoate, mesitoate, benzoate and the like. Carbonate hydroxy protecting groups include methyl vinyl, allyl, cinnamyl, benzyl and the like.

Detailed Description of the Embodiments

Various embodiments of the invention will now be described by way of illustration only with reference to the following Examples.

Example 1

Construction of building blocks

P3 building block

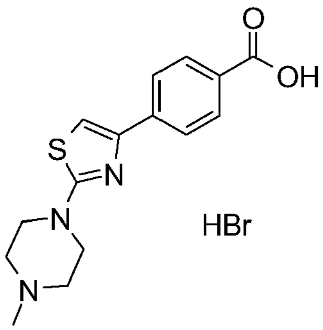

HBr

Step a) 4-Methyl-piperazine-1-carbothioic acid amide

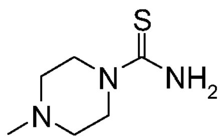

To a suspension of thiocarbonyldiimidazole (0.28 mol) in acetonitrile (300 ml) was added 1-methyl piperazine (0.28 mol). The reaction was stirred at RT for 1 h. The reaction was concentrated *in vacuo* to approximately half the volume and methanolic ammonia (7N, 0.7 mol) added. The reaction was heated at 55°C for 16 h. The reaction was concentrated *in vacuo* to approximately half the volume and cooled to 0°C at which point the product precipitated from solution. The product was collected by filtration to yield the title compound as a white solid (29.0 g). 1 H NMR (400MHz, $d_6$-DMSO) 2.15 (3H, s), 2.25-2.3 (4H, m), 3.71 (4H, s), 7.42 (2H,s).

Step b) 4-[2-(4-Methyl-piperazin-1-yl)-thiazol-4-yl]-benzoic acid hydrobromide

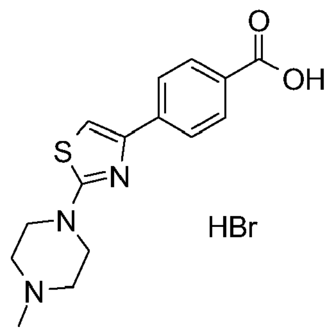

To a suspension of 4-methyl-piperazine-1-carbothioic acid amide (18.8 mmol) in ethanol (60 ml) was added 4-(2-bromo-acetyl)-benzoic acid (18.8 mmol). The reaction was heated at reflux for 1 h. The reaction was concentrated *in vacuo* to approximately half volume and the solid collected by filtration to yield the title compound as a white solid (15.8 mmol). 1H NMR (400MHz, $CD_3OD$) 2.97 (3H, s), 3.45 (4H, broad s), 3.95 (4H, broad s), 7.35 (1H, s), 7.97 (2H, d, J 8 Hz), 8.1 (2H, d, *J* 8Hz).

P1 building block

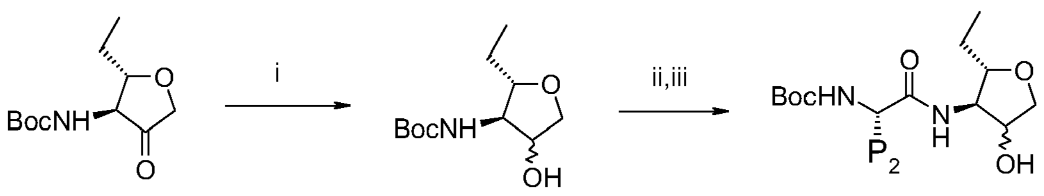


1 2

Scheme 3:

*Reagents and Conditions*: (i) $NaBH_4$, MeOH; (ii) 4MHCl/Dioxane (iii) WSC.HCl, DCM, NMM, Boc-P2-amino acid;

(2-(*S*)-Ethyl-4-oxo-tetrahydro-furan-3-(*S*)-yl)-carbamic acid *tert*-butyl ester (1) prepared as described in WO00/69855 (0.5 g, 2.2 mmol) was dissolved in methanol (5 mL) and cooled in an ice-bath. Sodium borohydride (91 mg, 2.4 mmol) was added and reaction stirred for 10 minutes. The ice-bath was removed and the reaction stirred for a further 1 h at room temperature. Water was added and the reaction was extracted with ethyl acetate and the organic layer washed with water. The organic layer was dried ($MgSO_4$) and the solvent removed *in vacuo* to give (2-(S)-ethyl-4-hydroxy-tetrahydro-furan-3-(*S*)-yl)-carbamic acid *tert*-butyl ester (2) as a white solid (338 mg, 67%). The structure was confirmed by NMR and MS analysis.

Compound (2) (338 mg, 1.5 mmol) was dissolved in 4M hydrochloric acid /dioxan and stirred at room temperature for 1 h. The solvent was removed in *vacuo* to give the hydrochloride salt as a white solid.

For solution phase elongation procedures, this hydrochloride salt can be suspended in dichloromethane (5 mL) and WSC.HCl (309 mg, 1.6 mmol) was added followed by the appropriate BOC-amino acid (517 mg, 1.8 mmol) and

NMM (321 uL, 2.9 mmol).

The completed P3-P2-P1 sequence is oxidised by Dess-Martin in DCM, for example 0.42 mmol P3-P2-P1 hydroxyl to which is added dichloromethane (2 mL) and Dess-Martin reagent (193 mg, 0.45 mmol).

Alternative P1 building block

C-5 Fluoroethylfuranone P1 building block

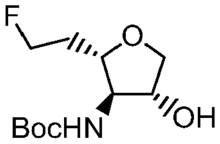

a) 3-Azido-3-deoxy-1,2-O-isopropylidene-alpha-D-galactofuranose

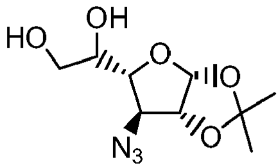

**(1)**

A mixture of 3-azido-3-deoxy-1,2:5,6-di-*O*-isopropylidene-alpha-D-galactofuranose (T. L. Lowary and O. Hindsgaul, Carbohydr. Res., 251 (1994) 33-67) (3.88 g, 13.6 mmol) in 50% aq. acetic acid (150 mL) was stirred at room temperature for 7 h, then stored at 8 °C overnight. The solution was then concentrated, re-dissolved in dichloromethane (50 mL), and washed with 1M sodium hydrogen carbonate (1x50 mL). The aqueous layer was extracted with dichloromethane (4x40 mL), and the extracts were combined, dried (sodium sulphate), filtered and concentrated *in vacuo.* Flash chromatography of the residue gave the title compound as a syrup (2.4g, 72%) which crystallized upon standing, and recovered starting material (0.7g, 18%).

b) 3-Azido-6-O-benzyl-3-deoxy-1,2-O-isopropylidene-alpha-D-galactofuranose

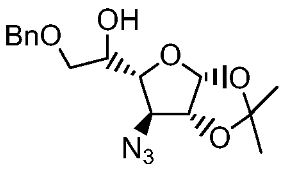

**(2)**

A mixture of diol **(1)** (13.5 g, 55 mmol) and dibutyltin oxide (13.7 g, 55 mmol) in toluene (300 mL) was refluxed for 2 h in a round bottomed flask equipped with a Dean-Stark trap. The solution was then concentrated *in vacuo*, and to the residue was added caesium fluoride (16.7 g, 110 mmol) and the solids were lyophilized for 2 h. The solid was then suspended in DMF (200 mL) and benzyl bromide (7.2 mL, 61 mmol) was added. The reaction mixture was stirred at room temperature overnight, and then partitioned between ethyl acetate (500 mL) and water (200 mL). The organic layer was then washed with water (3x200 mL), dried ($Na_2SO_4$), filtered and concentrated *in vacuo* onto silica. The product was purified by column chromatography (stepwise gradient elution, ethyl acetate in petroleum ether, 20-25%) to afford the title compound as a slightly yellow syrup of approximately 90% purity (14.7 g, 80%). $Delta_H$ (400 MHz, $CDCl_3$ at 298 K) 7.38-7.27 (5H, m, Ar-H), 5.86 (1H, d, *J* 3.9, H-1), 4.61 (1H, dd, $J_{2,3}$ 1.5, H-2), 4.57 (2H, d, $C\underline{H}_2Ph$), 4.19 (1H, m, H-3), 4.02-3.95 (2H, m, H-4 and H-5), 3.64 (1H, m, H-6b), 3.52 (1H, m, H-6a), 2.63 (1H, d, $J_{OH, H-5}$ 2.6, O<u>H</u>), 1.56 and 1.34 (6H, 2s, $C(C\underline{H}_3)_2$).

c) 3-Azido-6-*O*-benzyl-3,5-dideoxy-1,2-*O*-isopropylidene-beta-L-*arabino*-hexofuranose

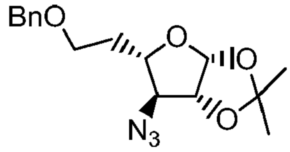

**(3)**

A solution of (2) (9.5 g, 28.3 mmol) and 1,1'-thiocarbonyldiimidazole (6.56 g, 36.8 mmol) in dichloromethane (100 mL) and pyridine (4.6 mL, 57 mmol) was stirred at room temperature overnight. The reaction mixture was then diluted with dichloromethane (100 mL), washed successively with 1M sulphuric acid (2x 100 mL) and 1M sodium hydrogen carbonate (1x100 mL), then dried ($Na_2SO_4$), filtered and concentrated *in vacuo.* The solid residue obtained was then re-dissolved in toluene (100 mL), heated to 80 °C, and then a solution of AIBN (2.79 g, 17 mmol) and tri-n-butyltin hydride (9.1 mL, 34 mmol) in toluene (80 mL) was added dropwise over approximately 40 min. The reaction was then stirred for another 40 min at 80 °C before being cooled and concentrated *in vacuo* onto silica. Flash chromatography of the residue with ethyl acetate in petroleum ether (stepwise gradient elution, 10-15%) gave the title compound as an oil (4.06 g, 45%).

d) 1,4-anhydro-3-azido-6-*O*-benzyl-3,5-dideoxy-L-*arabino*-hexitol

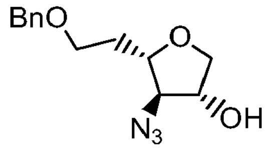

**(4)**

To a stirred solution of **(3)** (4.06 g, 12.7 mmol) in dichloromethane (180 mL) at 0 °C was added dropwise a solution of trimethylsilyl trifluoromethane-sulphonate (4.6 mL, 25.4 mmol) in dichloromethane (25 mL) over approximately 30 min. Then triethylsilane (10.1 mL, 63.6 mmol) was added and the reaction mixture was stirred at room temperature overnight. The reaction mixture was then washed with 1M sodium hydrogen carbonate (2x100 mL), and then concentrated *in vacuo* onto silica. Flash chromatography of the residue with ethyl acetate in petroleum ether (stepwise gradient elution, 20-50%) gave the title compound (4) as an oil (2.14 g, 64%).

d) 1,4-anhydro-6-*O*-benzyl-3[(*tert*-butoxycarbonyl)amino]-3,5-dideoxy-L-*arabino*-hexitol

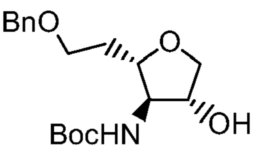

**(5)**

A mixture of (4) (0.78 g, 2.96 mmol), di-tert-butyl-dicarbonate (0.68 g, 3.11 mmol) and palladium on carbon (0.12 g, Acros, 10%) in 1:1 ethyl acetate-ethanol (20 mL), was hydrogenated at approximately atmospheric pressure for 3 h. The reaction mixture was filtered through Celite and concentrated *in vacuo.* Flash chromatography of the residue with ethyl acetate in petroleum ether (stepwise gradient elution, 20-50%) afforded the title compound (5) as a crystalline solid (0.89 g, 89%).

$Delta_H$ (400 MHz, $CDCl_3$ at 298 K) 7.38-7.27 (5H, m, Ar-H), 5.22 (1 H, br s, NH), 4.50 (2H, s, $C\underline{H_2}Ph$), 4.28 (1H, m, H-2), 4.00 (2H, m, H-1 b and OH), 3.82 (1H, dd, *J* 4.4, 9.8, H-1 a), 3.72 (1 H, m, H-4), 3.62 (2H, m, H-6a and H-6b), 3.51 (1H, m, H-3), 2.06 (1H, m, H-5b), 1.93 (1H, m, H-5a), 1.44 (9H, s, $C(C\underline{H_3})_3$).

e) 1,4-anhydro-2-*O*-benzoyl-3-[(*tert*-butoxycarbonyl)amino]-3,5-dideoxy-L-*arabino*-hexitol

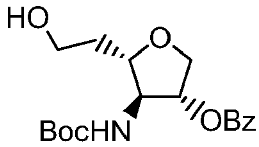
**(6)**

To a solution of **(5)** (1.02 g, 3.03 mmol) and dimethylaminopyridine (0.185 g, 1.51 mmol) in dichloromethane (20 mL) and pyridine (0.73 mL, 9.07 mmol) at 0 °C was added benzoyl chloride (0.39 mL, 3.33 mmol). The reaction mixture was stirred at room temperature for 4 h, then additional benzoyl chloride (0.2 mL) was added and the reaction mixture was stirred overnight. The reaction mixture was then diluted with dichloromethane (80 mL), washed successively with 1M sulphuric acid (2x50 mL) and 1M sodium hydrogen carbonate (1x50 mL), then dried ($Na_2SO_4$), filtered and concentrated *in vacuo.* Flash chromatography of the residue with ethyl acetate in petroleum ether (stepwise gradient elution, 20-40%) afforded a solid (1.25 g, 94%). A mixture of the benzoate (1.23 g, 2.79 mmol) and palladium on charcoal (0.1 g, Acros, 10%) in 2:1 ethyl acetate-ethanol (30 mL) was hydrogenated at approximately atmospheric pressure for 90 min, then filtered through Celite and concentrated *in vacuo* to afford a crystalline solid (0.98 g, quantitative).
$Delta_H$ (400 MHz, $CDCl_3$ at 298 K) 8.04 (2H, m, Ar-H), 7.58 (1H, m, Ar-H), 7.46 (2H, m, Ar-H), 5.34 (1H, m, H-2), 4.95 (1H, d, NH), 4.16 (1H, dd, J 5.4, 10.7, H-1b), 4.06 (2H, m, H-1a and H-3), 3.94 (1H, m, H-3), 3.85 (2H, m, H-6a and H-6b), 2.85 (1H, br s, OH), 2.02 (2H, m, H-5a and H-5b), 1.44 (9H, s, $C(C\underline{H}_3)_3$).

f) 1,4-anhydro-3-[(*tert*-butoxycarbonyl)amino]-3,5,6-trideoxy-6-fluoro-L-*arabino*-hexitol

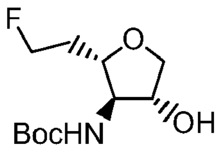
**(7)**

To a solution of **(6)** (0.50 g, 1.42 mmol) in dichloromethane at 0 °C was added bis-(2-methoxyethyl)aminosulphurtrifluoride (0.39 mL, 2.13 mmol). The reaction mixture was stirred at room temperature overnight, then diluted with dichloromethane (40 mL), washed with 1M sodium hydrogen carbonate (2x30 mL), then dried ($Na_2SO_4$), filtered and concentrated *in vacuo* onto silica. Flash chromatography of the residue with ethyl acetate in petroleum ether (stepwise gradient elution, 20-40%) afforded a syrup (0.31 g, 62%). This was then treated with 0.25M methanolic sodium methoxide (6 mL) at room temperature for 45 min, neutralized with Dowex $H^+$resin and filtered. The filtrate was made alkaline with triethylamine and concentrated *in vacuo.* Column chromatography of the residue with ethyl acetate in petroleum ether (stepwise gradient elution, 40-60%) gave a crystalline solid (0.19 g, 85%).
$Delta_H$ (400 MHz, $CDCl_3$ at 298 K) 4.74 (1H, br s, NH), 4.66 (1H, m, H-6b), 4.54 (1H, m, H-6a), 4.29 (1H, m, H-2), 4.00 (1H, dd, *J* 6.3, 9.8, H-1 b), 3.84 (1H, dd, *J* 3.9, 9.8, H-1a), 3.74 (1H, m, H-4), 3.62 (1H, m, H-3), 3.52 (1H, br s, OH), 2.06 (2H, m, H-5a and H-5b), 1.46 (9H, s, $C(C\underline{H}_3)_3$).

<u>Alternative P1 building block</u>

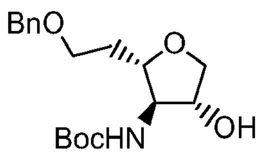

a) 3-Azido-3-deoxy-1,2-*O*-isopropylidene-alpha-D-galactofuranose

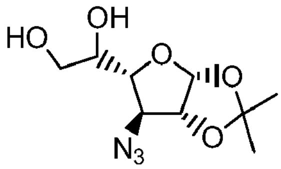

(1)

A mixture of 3-azido-3-deoxy-1,2:5,6-di-O-isopropylidene-alpha-D-galactofuranose (T. L. Lowary and O. Hindsgaul, Carbohydr. Res., 251 (1994) 33-67) (3.88 g, 13.6 mmol) in 50% aq. acetic acid (150 mL) was stirred at room temperature for 7 h, then stored at 8 °C overnight. The solution was then concentrated, re-dissolved in dichloromethane (50 mL), and washed with 1M sodium hydrogen carbonate (1x50 mL). The aqueous layer was extracted with dichloromethane (4x40 mL), and the extracts were combined, dried (sodium sulphate), filtered and concentrated *in vacuo.* Flash chromatography of the residue gave the title compound as a syrup (2.4g, 72%) which crystallized upon standing, and recovered starting material (0.7g, 18%).

b) 3-Azido-6-*O*-benzyl-3-deoxy-1,2-*O*-isopropylidene-alpha-D-galactofuranose

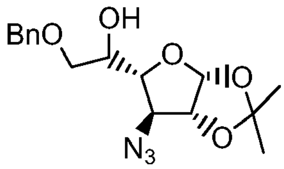

**(2)**

A mixture of diol (1) (13.5 g, 55 mmol) and dibutyltin oxide (13.7 g, 55 mmol) in toluene (300 mL) was refluxed for 2 h in a round bottomed flask equipped with a Dean-Stark trap. The solution was then concentrated *in vacuo*, and to the residue was added caesium fluoride (16.7 g, 110 mmol) and the solids were lyophilized for 2 h. The solid was then suspended in DMF (200 mLl) and benzyl bromide (7.2 mL, 61 mmol) was added. The reaction mixture was stirred at room temperature overnight, and then partitioned between ethyl acetate (500 mL) and water (200 mL). The organic layer was then washed with water (3x200 mL), dried ($Na_2SO_4$), filtered and concentrated *in vacuo* onto silica. The product was purified by column chromatography (stepwise gradient elution, ethyl acetate in petroleum ether, 20-25%) to afford the title compound as a slight yellow syrup of approximately 90% purity (14.7 g, 80%).

$\text{Delta}_H$ (400 MHz, $CDCl_3$ at 298 K) 7.38-7.27 (5H, m, Ar-H), 5.86 (1H, d, *J* 3.9, H-1), 4.61 (1H, dd, $J_{2,3}$ 1.5, H-2), 4.57 (2H, d, C<u>H</u>$_2$Ph), 4.19 (1H, m, H-3), 4.02-3.95 (2H, m, H-4 and H-5), 3.64 (1H, m, H-6b), 3.52 (1H, m, H-6a), 2.63 (1H, d, $J_{OH,\ H\text{-}5}$ 2.6, O<u>H</u>), 1.56 and 1.34 (6H, 2s, C(C<u>H</u>$_3$)$_2$).

c) 3-Azido-6-*O*-benzyl-3,5-dideoxy-1,2-*O*-isopropylidene-beta-L-*arabino*-hexofuranose

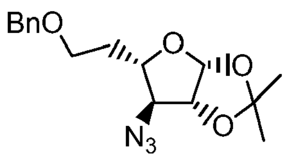

(3)

A solution of (2) (9.5 g, 28.3 mmol) and 1,1'-thiocarbonyldiimidazole (6.56 g, 36.8 mmol) in dichloromethane (100 mL) and pyridine (4.6 mL, 57 mmol) was stirred at room temperature overnight. The reaction mixture was then diluted with dichloromethane (100 mL), washed successively with 1M sulphuric acid (2x 100 mL) and 1M sodium hydrogen carbonate (1x100 mL), then dried ($Na_2SO_4$), filtered and concentrated *in vacuo.* The solid residue obtained was then re-dissolved in toluene (100 mL), heated to 80 °C, and then a solution of AIBN (2.79 g, 17 mmol) and tri-n-butyltin hydride (9.1 mL, 34 mmol) in toluene (80 mL) was added dropwise over approximately 40 min. The reaction was then stirred for another 40 min at 80 °C before being cooled and concentrated *in vacuo* onto silica. Flash chromatography of the residue with ethyl acetate in petroleum ether (stepwise gradient elution, 10-15%) gave the title compound as an oil (4.06 g, 45%).

d) 1,4-anhydro-3-azido-6-O-benzyl-3,5-dideoxy-L-*arabino*-hexitol

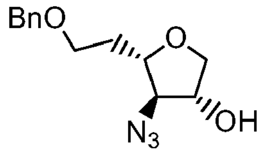
(4)

To a stirred solution of (3) (4.06 g, 12.7 mmol) in dichloromethane (180 mL) at 0 °C was added dropwise a solution of trimethylsilyl trifluoromethane-sulphonate (4.6 mL, 25.4 mmol) in dichloromethane (25 mL) over approximately 30 min. Then triethylsilane (10.1 mL, 63.6 mmol) was added and the reaction mixture was stirred at room temperature overnight. The reaction mixture was then washed with 1M sodium hydrogen carbonate (2x100 mL), and then concentrated *in vacuo* onto silica. Flash chromatography of the residue with ethyl acetate in petroleum ether (stepwise gradient elution, 20-50%) gave the title compound (4) as an oil (2.14 g, 64%).

d) 1,4-anhydro-6-*O*-benzyl-3[(*tert*-butoxycarbonyl)amino]-3,5-dideoxy-L-*arabino*-hexitol

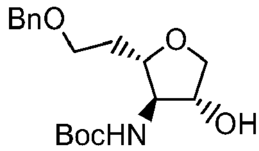
(5)

A mixture of (4) (0.78 g, 2.96 mmol), di-tert-butyl-dicarbonate (0.68 g, 3.11 mmol) and palladium on carbon (0.12 g, Acros, 10%) in 1:1 ethyl acetate-ethanol (20 mL), was hydrogenated at approximately atmospheric pressure for 3 h. The reaction mixture was filtered through celite and concentrated *in vacuo.* Flash chromatography of the residue with ethyl acetate in petroleum ether (stepwise gradient elution, 20-50%) afforded the title compound (5) as a crystalline solid (0.89 g, 89%).

Delta$_H$ (400 MHz, $CDCl_3$ at 298 K) 7.38-7.27 (5H, m, Ar-H), 5.22 (1H, br s, NH), 4.50 (2H, s, C$\underline{H}$2Ph), 4.28 (1H, m, H-2), 4.00 (2H, m, H-1b and OH), 3.82 (1H, dd, *J* 4.4, 9.8, H-1a), 3.72 (1H, m, H-4), 3.62 (2H, m, H-6a and H-6b), 3.51 (1H, m, H-3), 2.06 (1H, m, H-5b), 1.93 (1H, m, H-5a), 1.44 (9H, s, C(C$\underline{H}_3$)$_3$).

As with other hydroxylated P1 building blocks, the completed P3-P2-P1 sequence is typically oxidised to the active ketone. The $R^1$ protecting group (in this case Bn is typically done aftewards by hydrogenation.

Example 2

N-[1-(2-Ethyl-4-oxo-tetrahydro-furan-3-ylcarbamoyl)-cyclohexyl]-4-[2-(4-methylpiperazin-1-yl)-thiazol-4-yl]-benzamide

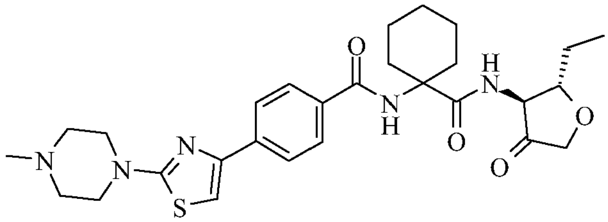

The compound was prepared as outlined in Scheme 4.

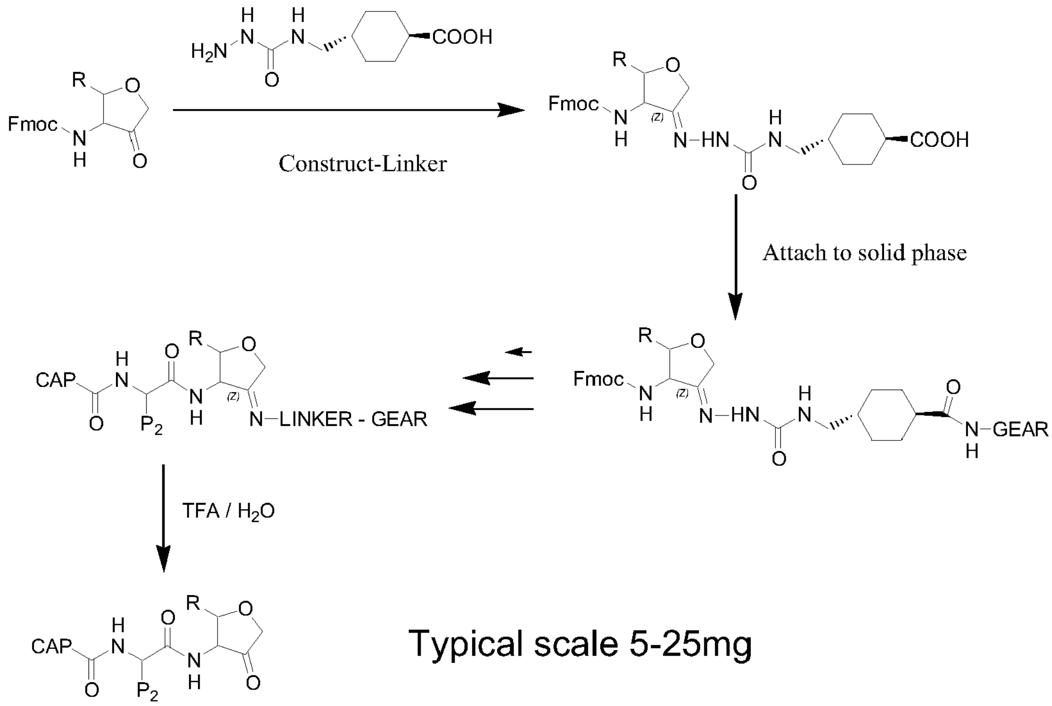


Scheme 4. Solid phase synthesis of dihydro-2(3H)-5-alkyl furanone inhibitors of cathepsin.

Preparation of the P1 building block and loading of the solid phase was as described in WO02/088106 and WO00/69855. Acylation of the sterically hindered spirocycle building block (5eq) was carried out using HBTU (5eq), HOBt (5eq), NMM (10eq) in DMF for 6 hrs and then repeated with fresh reagents for 18hrs. Acylation of the piperazine acid of Example 1 (3eq) was carried out using HBTU (3eq), HOBt (3eq), NMM (6eq) in DMF for 18 hrs. Release from the resin and generation of the ketone was as described in WO00/69855.

Reverse phase high-pressure chromatography (RP-HPLC) was performed using dual wavelength detection of 214/254nm using a Phenomonex analytical Synergi Max-RP (4u, 50 x 4.6 mm) column. Chromatographic separations were achieved using a linear gradient (10-90%) of buffer B in A (A=0.1 % aqueous TFA; B=90% MeCN, 10% A) over 10 min at a flow rate of 2ml/min.

RPHPLC-electrospray mass spectrometry (ES-MS) analysis was conducted through Waters 2795 injection module onto a Phenomenex Synergi Max-RP $C_{12}$ (4μ, 50 x 2.0 mm) column. Gradient: 10-90% B in A over 10 min, 500 ul/min, where solvent A was 0.1% aq TFA and solvent B was 90% acetonitrile/10% water/0.1 % TFA. Eluent flow from the column was diverted through a Waters 2487 UV absorbance detector measured at 215nm and continuously monitored *via* a Waters ZQ mass spectrometer (m/z) (electrospray positive, ES+ and negative ES- mode).

Purification was achieved with semi-prep HPLC using gradient elution with two mobile phases (0.1 % aq TFA and MeCN). Lyophilisation of the appropriate fractions isolated the final product as a white solid.

White solid (36 mg, 9.1%) overall yield based on furanone loading on solid phase HPLC: Peak 1 $R_t$ 3.14min (98%). *m*/*z*(ESMS) 540.16 (M+H 31.0%).

Example 3

N-[1-(2-Ethyl-4-oxo-tetrahydro-furan-3-ylcarbamoyl)-3-methyl-butyl]-4-[2-(4-methylpiperazin-1-yl)-thiazol-4-yl]-benzamide

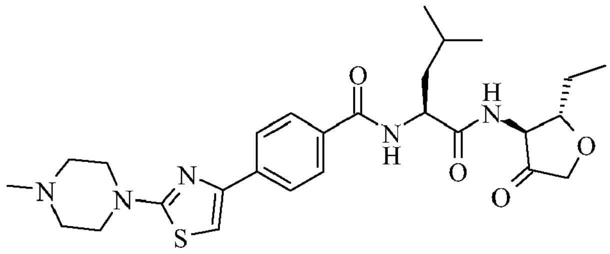

The compound was synthesised as shown in Example 2, but acylation of L-leucine was carried out with the reagents described in just one 18h coupling.

Purification was achieved with semi-prep HPLC using gradient elution with two mobile phases (0.1 % aq TFA and MeCN). Lyophilisation of the appropriate fractions isolated the final product as a white solid.

White solid (76 mg, 23.4%) overall yield based on furanone loading on solid phase. HPLC: Peak 1 $R_t$ 3.24min (98%). *m*/*z*(ESMS) 528.12 (M+H 67.0%).

Biological Examples

Determination of cathepsin K Proteolytic catalytic activity

Convenient assays for cathepsin K are carried out using human recombinant enzyme, such as that described in PDB.
ID BC016058 standard; mRNA; HUM; 1699 BP.
DE Homo sapiens cathepsin K (pycnodysostosis), mRNA (cDNA clone
MGC:23107
RX MEDLINE;. RX PUBMED; 12477932.
DR RZPD; IRALp962G1234.
DR SWISS-PROT; P43235;

The recombinant cathepsin K can be expressed in a variety of commercially available expression systems including E coli, Pichia and Baculovirus systems. The purified enzyme is activated by removal of the prosequence by conventional methods.

Standard assay conditions for the determination of kinetic constants used a fluorogenic peptide substrate, typically H-D-Ala-Leu-Lys-AMC, and were determined in either 100 mM Mes/Tris, pH 7.0 containing 1 mM EDTA and 10 mM 2-mercaptoethanol or 100 mM Na phosphate, 1 mM EDTA, 0.1 %PEG4000 pH 6.5 or 100 mM Na acetate, pH 5.5 containing 5 mM EDTA and 20 mM cysteine, in each case optionally with 1M DTT as stabiliser. The enzyme concentration used was 5 nM. The stock substrate solution was prepared at 10 mM in DMSO. Screens were carried out at a fixed substrate concentration of 60 uM and detailed kinetic studies with doubling dilutions of substrate from 250 uM. The total DMSO concentration in the assay was kept below 3%. All assays were conducted at ambient temperature. Product fluorescence (excitation at 390 nm, emission at 460 nm) was monitored with a Labsystems Fluoroskan Ascent fluorescent plate reader. Product progress curves were generated over 15 minutes following generation of AMC product.

Cathepsin S Ki determination

The assay uses baculovirus-expressed human cathepsin S and the boc-Val-Leu-Lys-AMC fluorescent substrate available from Bachem in a 384 well plate format, in which 7 test compounds can be tested in parallel with a positive control comprising a known cathepsin S inhibitor comparator.

Substrate dilutions

280 ul/well of 12.5% DMSO are added to rows B - H of two columns of a 96 deep well polypropylene plate. 70 ul/well of substrate is added to row A. 2 x 250 ul/well of assay buffer (100 mM Na phosphate, 100 mM NaCl, pH 6.5) is added to row A, mixed, and double diluted down the plate to row H.

Inhibitor dilutions.

100 ul/well of assay buffer is added to columns 2-5 and 7-12 of 4 rows of a 96 well V bottom polypropylene

plate. 200 ul/well of assay buffer is added to columns 1 and 6.

The first test compound prepared in DMSO is added to column 1 of the top row, typically at a volume to provide between 10 and 30 times the initially determined rough $K_i$. The rough Ki is calculated from a preliminary run in which 10 ul/well of 1mM boc-VLK-AMC (1/10 dilution of 10 mM stock in DMSO diluted into assay buffer) is dispensed to rows B to H and 20 ul/well to row A of a 96 well Microfluor ™ plate. 2 ul of each 10 mM test compound is added to a separate well on row A, columns 1-10. Add 90 ul assay buffer containing 1 mM DTT and 2 nM cathepsin S to each well of rows B-H and 180 ul to row A. Mix row A using a multichannel pipette and double dilute to row G. Mix row H and read in the fluorescent spectrophotometer. The readings are Prism data fitted to the competitive inhibition equation, setting S = 100 uM and $K_M$ = 100 uM to obtain an estimate of the $K_i$, up to a maximum of 100 uM.

The second test compound is added to column 6 of the top row, the third to column 1 of the second row etc. Add 1 ul of comparator to column 6 of the bottom row. Mix column 1 and double dilute to column 5. Mix column 6 and double dilute to column 10.

Using an 8-channel multistepping pipette set to 5 x 10 ul, distribute 10 ul/well of substrate to the 384 well assay plate. Distribute the first column of the substrate dilution plate to all columns of the assay plate starting at row A. The tip spacing of the multichannel pipette will correctly skip alternate rows. Distribute the second column to all columns starting at row B.

Using a 12-channel multistepping pipette set to 4 x 10 ul, distribute 10 ul/well of inhibitor to the 384 well assay plate. Distribute the first row of the inhibitor dilution plate to alternate rows of the assay plate starting at A1. The tip spacing of the multichannel pipette will correctly skip alternate columns. Similarly, distribute the second, third and fourth rows to alternate rows and columns starting at A2, B1 and B2 respectively.

Mix 20 ml assay buffer and 20 ul 1 M DTT. Add sufficient cathepsin S to give 2 nM final concentration.

Using the a distributor such as a Multidrop 384, add 30 ul/well to all wells of the assay plate and read in fluorescent spectrophotometer such as an Ascent.

Fluorescent readings, (excitation and emission wavelengths 390nm and 460nm respectively, set using bandpass filters) reflecting the extent of enzyme cleavage of the fluorescent substrate, notwithstanding the inhibitor, are linear rate fitted for each well.

Fitted rates for all wells for each inhibitor are fitted to the competitive inhibition equation using SigmaPlot 2000 to determine V, Km and Ki values.

Cathepsin L Ki

The procedure above with the following amendments is used for the determination of Ki for cathepsin L.

The enzyme is commercially available human cathepsin L (for example Calbiochem). The substrate is H-D-Val-Leu-Lys-AMC available from Bachem. The assay buffer is 100 mM sodium acetate 1 mM EDTA, pH5.5) The DMSO stock (10 mM in 100%DMSO) is diluted to 10% in assay buffer. Enzyme is prepared at 5 nM concentration in assay buffer plus 1 mM dithiothreitol just before use. 2 ul of 10 mM inhibitor made up in 100% DMSO is dispensed into row A. 10 ul of 50 uM substrate (=1/200 dilution of 10 mM stock in DMSO, diluted in assay buffer)

Inhibition Studies

Potential inhibitors are screened using the above assay with variable concentrations of test compound. Reactions were initiated by addition of enzyme to buffered solutions of substrate and inhibitor. $K_i$ values were calculated according to equation 1

$$v_0 = \frac{VS}{K_M\left(1+\frac{I}{K_i}\right)+S} \quad (1)$$

where $v_0$ is the velocity of the reaction, $V$ is the maximal velocity, $S$ is the concentration of substrate with Michaelis constant of $K_M$, and $I$ is the concentration of inhibitor.

Representative compounds of the invention were assayed for cathepsin K potency and selectivity in the assays above. Note that the cathepsin L and S data is in micromolar, whereas the compounds are so potent against cathepsin

K that the results are presented in nanomolar.

| | Cathepsin K Ki | Cathepsin L Ki | Cathepsin S Ki |
|---|---|---|---|
| Example 2 | 4 nM | 120 uM | 770 uM |
| Example 3 | 1 nM | 0.5 uM | 1.8 uM |

It will be apparent that these compounds of the invention are very potent against cathepsin K but also show at least 100 fold selectivity against the closely related cysteine proteases cathepsin L and S. Additionally, the compounds typically possess good permeability (as measured below) and other DMPK properties.

Permeability

This example measures transport of inhibitors through the cells of the human gastroenteric canal. The assay uses the well known Caco-2 cells with a passage number between 40 and 60.

Apical to basolateral transport

Generally every compound will be tested in 2-4 wells. The basolateral and the apical wells will contain 1.5 mL and 0.4 mL transport buffer (TB), respectively, and the standard concentration of the tested substances is 10 uM. Furthermore all test solutions and buffers will contain 1% DMSO. Prior to the experiment the transport plates are pre-coated with culture medium containing 10% serum for 30 minutes to avoid nonspecific binding to plastic material. After 21 to 28 days in culture on filter supports the cells are ready for permeability experiments.

Transport plate no 1 comprises 3 rows of 4 wells each. Row 1 is denoted Wash, row 2 "30 minutes" and row 3 "60 minutes". Transport plate no 2 comprises 3 rows of 4 wells, one denoted row 4 "90 minutes", row 5 "120 minutes and the remaining row unassigned.

The culture medium from the apical wells is removed and the inserts are transferred to a wash row (No. 1) in a transport plate (plate no.1) out of 2 plates without inserts, which have already been prepared with 1.5 mL transport buffer (HBSS, 25 mM HEPES, pH 7.4) in rows 1 to 5. In A→B screening the TB in basolateral well also contains 1% Bovine Serum Albumin.

0.5 mL transport buffer (HBSS, 25 mM MES, pH 6.5) is added to the inserts and the cell monolayers equilibrated in the transport buffer system for 30 minutes at 37 °C in a polymix shaker. After being equilibrated to the buffer system the Transepithelial electrical resistance value (TEER) is measured in each well by an EVOM chop stick instrument. The TEER values are usually between 400 to 1000 Ω per well (depends on passage number used).

The transport buffer (TB, pH 6.5) is removed from the apical side and the insert is transferred to the 30 minutes row (No. 2) and fresh 425 uL TB (pH 6.5), including the test substance is added to the apical (donor) well. The plates are incubated in a polymix shaker at 37°C with a low shaking velocity of approximately 150 to 300 rpm.

After 30 minutes incubation in row 2 the inserts will be moved to new prewarmed basolateral (receiver) wells every 30 minutes; row 3 (60 minutes), 4 (90 minutes) and 5 (120 minutes).

25 uL samples will be taken from the apical solution after ~2 minutes and at the end of the experiment. These samples represent donor samples from the start and the end of the experiment.

300 uL will be taken from the basolateral (receiver) wells at each scheduled time point and the post value of TEER is measured at the end the experiment. To all collected samples acetonitrile will be added to a final concentration of 50% in the samples. The collected samples will be stored at -20°C until analysis by HPLC or LC-MS.

Basolateral to apical transport

Generally every compound will be tested in 2-4 wells. The basolateral and the apical wells will contain 1.55 mL and 0.4 mL TB, respectively, and the standard concentration of the tested substances is 10 uM. Furthermore all test solutions and buffers will contain 1% DMSO. Prior to the experiment the transport plates are precoated with culture medium containing 10% serum for 30 minutes to avoid nonspecific binding to plastic material.

After 21 to 28 days in culture on filter supports the cells are ready for permeability experiments. The culture medium from the apical wells are removed and the inserts are transferred to a wash row (No.1) in a new plate without inserts (Transport plate).

The transport plate comprises 3 rows of 4 wells. Row 1 is denoted "wash" and row 3 is the "experimental row". The transport plate has previously been prepared with 1.5 mL TB (pH 7.4) in wash row No. 1 and with 1.55 mL TB (pH 7.4), including the test substance, in experimental row No. 3 (donor side).

0.5 mL transport buffer (HBSS, 25 mM MES, pH 6.5) is added to the inserts in row No. 1 and the cell monolayers are equilibrated in the transport buffer system for 30 minutes, 37 °C in a polymix shaker. After being equilibrated to the buffer system the TEER value is measured in each well by an EVOM chop stick instrument.

The transport buffer (TB, pH 6.5) is removed from the apical side and the insert is transferred to row 3 and 400 uL fresh TB, pH 6.5 is added to the inserts. After 30 minutes 250 uL is withdrawn from the apical (receiver) well and replaced by fresh transport buffer. Thereafter 250 uL samples will be withdrawn and replaced by fresh transport buffer every 30 minutes until the end of the experiment at 120 minutes, and finally a post value of TEER is measured at the end of the experiment. A 25 uL samples will be taken from the basolateral (donor) compartment after ~2 minutes and at the end of the experiment. These samples represent donor samples from the start and the end of the experiment.

To all collected samples acetonitrile will be added to a final concentration of 50% in the samples. The collected samples will be stored at -20°C until analysis by HPLC or LC-MS.

Calculation

Determination of the cumulative fraction absorbed, $FA_{cum}$, versus time. $FA_{cum}$ is calculated from:

$$FA_{cum} = \sum \frac{C_{RI}}{C_{DI}}$$

Where $C_{Ri}$ is the receiver concentration at the end of the interval i and $C_{Di}$ is the donor concentration at the beginning of interval i. A linear relationship should be obtained.

The determination of permeability coefficients ($P_{app}$, cm/s) are calculated from:

$$P_{app} = \frac{(k \cdot V_R)}{(A \cdot 60)}$$

where k is the transport rate ($min^{-1}$) defined as the slope obtained by linear regression of cumulative fraction absorbed ($FA_{cum}$) as a function of time (min), $V_R$ is the volume in the receiver chamber (mL), and A is the area of the filter ($cm^2$).

Reference compounds

| Category of absorption in man | Markers | % absorption in man |
|---|---|---|
| PASSIVE TRANSPORT | | |
| Low (0-20%) | Mannitol<br>Methotrexate | 16<br>20 |
| Moderate (21-75%) | Acyclovir | 30 |
| High (76-100%) | Propranolol<br>Caffeine | 90<br>100 |
| ACTIVE TRANSPORT | | |
| Amino acid transporter | L-Phenylalanine | 100 |
| ACTIVE EFFLUX | | |
| PGP-MDR1 | Digoxin | 30 |

| | Papp |
|---|---|
| Example 2 | 36 |
| Example 3 | 46 |
| Prior art | 1 |

The prior art compound is the N-methylpiperazinenbenzoic acid derivative appearing on page 91, line 13 of WO02/88106, whose structure appears in the Background above. It will be apparent that the compounds of the invention produce very favourable permeability values in comparison to the marginal permeability of the prior art.

All references referred to in this application, including patents and patent applications, are incorporated herein by reference to the fullest extent possible.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

## Claims

1. A compounds of the Formula II

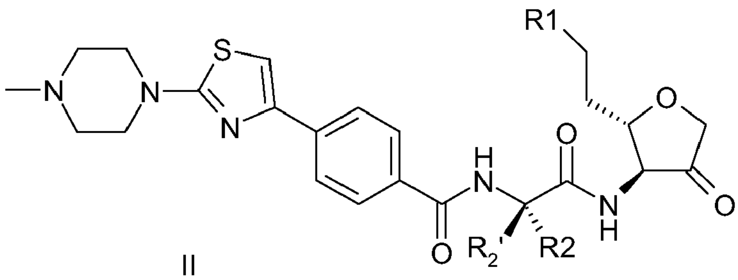

II

wherein
$R^1$ is H, OH, F
$R^2$ is $-CH_2CH(CH_3)_2$, $-CHC(CH_2)_3$ or $-CH(CH^3)(CH_2CH_3)$;
$R^{2'}$ is H; or
$R^2$ and $R^{2'}$ together with the adjacent C atom defines cyclohexyl;
and pharmaceutically acceptable salts, hydrates and N-oxides thereof.

2. A compound of the Formula II, wherein $R^1$ is H.

3. A compound according to any preceding claim wherein $R^{2'}$ is H and $R^2$ is $CH_2C(CH_3)_2$.

4. A compound according to claim 1 or 2 wherein $R^{2'}$ and $R^2$, together with the carbon atom to which they are attached define a cyclohexyl group.

5. A pharmaceutical composition comprising a compound as defined in any of claims 1 to 4 and a pharmaceutically acceptable carrier or diluent therefor.

6. Use of a compound as defined in any of claims 1 to 4 in the manufacture of a medicament for the treatment of disorders **characterised by** inappropriate expression or activation of cathepsin K.

7. Use according to claim 6, wherein the disorder is selected from:

    osteoporosis,
    gingival diseases such as gingivitis and periodontitis,

Paget's disease,
hypercalcaemia of malignancy
metabolic bone disease
diseases **characterised by** excessive cartilage or matrix degradation,
such as osteoarthritis and rheumatoid arthritis.
bone cancers including neoplasia,
pain.

**8.** A compound as defined in any of claims 1 to 4 for use as a medicament.

**9.** A compound as defined in any one of claims 1 to 4 for use in the treatment or prevention of a disorder **characterised by** inappropriate expression or activation of cathepsin K.

**10.** A method for the treatment or prevention of a disorder **characterised by** inappropriate expression or activation of cathepsin K comprising the administration of a safe and effective amount of a compound according to any one of claims 1 to 4 to a subject in need thereof.

European Patent Office

# PARTIAL EUROPEAN SEARCH REPORT

which under Rule 45 of the European Patent Convention shall be considered, for the purposes of subsequent proceedings, as the European search report

Application Number
EP 06 12 2877

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 02/088106 A (MEDIVIR AB [SE]) 7 November 2002 (2002-11-07) * the whole document * ----- | 1-10 | INV. C07D417/12 A61K31/495 A61P35/00 A61P19/00 C07K5/06 |
| A | WO 00/69855 A (MEDIVIR UK LTD [GB]; PEPTIMMUNE INC [US]; QUIBELL MARTIN [GB]; TAYLOR) 23 November 2000 (2000-11-23) * the whole document * ----- | 1-10 | |

TECHNICAL FIELDS SEARCHED (IPC)

C07D
A61K
A61P
C07K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| The Hague | 23 November 2006 | Fritz, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................
& : member of the same patent family, corresponding document

EPO FORM 1503 03.82 (P04C07)

European Patent Office

INCOMPLETE SEARCH SHEET C

Application Number
EP 06 12 2877

Although claim 10 is directed to a method of treatment of the human (Article 52(4) EPC), the search has been carried out and based on the alleged effects of the compounds.

-----

## ANNEX TO THE EUROPEAN SEARCH REPORT ON EUROPEAN PATENT APPLICATION NO. EP 06 12 2877

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2006

| Patent document cited in search report | | Publication date | Patent family member(s) | | | Publication date |
|---|---|---|---|---|---|---|
| WO 02088106 | A | 07-11-2002 | AT | 323083 | T | 15-04-2006 |
| | | | CA | 2429001 | A1 | 07-11-2002 |
| | | | EP | 1358183 | A2 | 05-11-2003 |
| | | | JP | 2004520439 | T | 08-07-2004 |
| WO 0069855 | A | 23-11-2000 | AT | 260274 | T | 15-03-2004 |
| | | | AU | 763694 | B2 | 31-07-2003 |
| | | | AU | 4772200 | A | 05-12-2000 |
| | | | BR | 0010553 | A | 02-07-2002 |
| | | | CA | 2374297 | A1 | 23-11-2000 |
| | | | DE | 60008524 | D1 | 01-04-2004 |
| | | | DE | 60008524 | T2 | 23-12-2004 |
| | | | DK | 1178986 | T3 | 05-07-2004 |
| | | | EP | 1178986 | A2 | 13-02-2002 |
| | | | ES | 2215048 | T3 | 01-10-2004 |
| | | | IL | 146409 | A | 20-11-2005 |
| | | | JP | 2002544274 | T | 24-12-2002 |
| | | | MX | PA01011872 | A | 04-09-2003 |
| | | | PT | 1178986 | T | 30-07-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0288106 A
- WO 200664286 A
- WO 0069855 A
- WO 02057270 A
- WO 0664286 A
- WO 02088106 A


### Non-patent literature cited in the description


- **FULLER, LAWRENCE ROSS ; GRABOWSKA SHIROO.** Samuelsson and Chambers. *J. Clin. Invest.*
- **JEAN JACQUES ANDRE COLLET ; SAMUEL H. WILEN.** Enantiomers, Racemates and Resolutions. John Wiley & Sons, Inc, 1981
- **M. BODANSZKY.** Peptide Chemistry. Springer-Verlag, 1993
- **GREENE.** Protective Groups in Organic Chemistry. John Wiley & Sons, 1981
- The Peptides: Analysis, Synthesis, Biology. Academic Press, 1981, vol. 3
- **GREENE.** Protective Groups in Organic Synthesis. John Wiley & Sons, 1981
- **T. L. LOWARY ; O. HINDSGAUL.** *Carbohydr. Res.,* 1994, vol. 251, 33-67
- **T. L. LOWARY ; O. HINDSGAUL.** *Carbohydr. Res,* 1994, vol. 251, 33-67